Europäisches Patentamt

(19) European Patent Office

Office européen des brevets

(11) Numéro de publication: **0117164**
**B1**

(12) ## FASCICULE DE BREVET EUROPEEN

(45) Date de publication du fascicule du brevet:
**07.01.87**

(51) Int. Cl.⁴: **A 61 K 9/54,** A 61 K 9/00

(21) Numéro de dépôt: **84400032.3**

(22) Date de dépôt: **06.01.84**

(54) **Médicament à libération programmée associant l'acide acétylsalicylique à la dihydroergotamine.**

(30) Priorité: **28.01.83 FR 8301577**

(43) Date de publication de la demande:
**29.08.84 Bulletin 84/35**

(45) Mention de la délivrance du brevet:
**07.01.87 Bulletin 87/2**

(84) Etats contractants désignés:
**AT BE CH DE FR GB IT LI LU**

(56) Documents cités:
**EP - A - 0 036 350**
**EP - A - 0 064 485**
**FR - A - 2 444 460**
**FR - A - 2 453 640**
**FR - A - 2 454 804**
**GB - A - 2 047 096**

**BIOLOGICAL ABSTRACTS, vol. 74, no. 10, 1982, résumé 70382, Philadelphia, PA, US, F. ZEKERT et al: "Acetylsalicyl acid in combination with dihydroergotamine for preventing thromboembolism" F. ZEKERT et coll., Haemostasis 11, 149-153, 1982**

**Le dossier contient des informations techniques présentées postérieurement au dépôt de la demande et ne figurant pas dans le présent fascicule.**

(73) Titulaire: **SANOFI, Société dite:, 40, Avenue George V, F-75008 Paris (FR)**

(72) Inventeur: **Chick, Jacques Alexandre, 2 Rue des Fontaines, F-92310 Sevres (FR)**

(74) Mandataire: **Poius, Camille et al, c/o Cabinet Lavoix 2, Place d'Estienne d'Orves, F-75441 Paris Cedex 09 (FR)**

## Description

Des travaux expérimentaux ont montré l'intérêt de l'administration simultanée de comprimés de dihydroergotamine (DHE) et de comprimés d'acide acétylsalicylique (ASA) dans la prévention des thrombo-embolies (ZEKERT et al., HAEMOSTASIS, 1982, 11, 149-153) à des doses unitaires de 5 mg de DHE et de 500 mg d'ASA, le traitement étant poursuivi pendant plusieurs jours (1 à 2 semaines). Néanmoins, les auteurs ont exclu de leur essai les patients ne pouvant supporter les effets indésirables de la DHE ou de l'ASA. En effet, L'ASA peut provoquer, en particulier aux doses utilisées par ZEKERT, des saignements digestifs occultes et des hémorragies.

Etant très peu soluble en phase aqueuse, il se disperse peu dans le contenu gastrique. Il en résulte de fortes concentrations en un petit nombre de points de la muqueuse gastrique, ce qui favorise l'apparition d'une gastrite hémorragique. Certes, celle-ci est d'une pathogénie complexe, mais pour l'essentiel, elle résulte d'un effet direct de contact de l'ASA avec la muqueuse gastrique.

C'est la raison pour laquelle différentes formes galéniques ont été proposées pour éviter ces inconvénients et améliorer la tolérance digestive de l'ASA.

C'est ainsi que sont commercialisés des comprimés entériques, des comprimés à enrobage gastrorésistant et entéro-soluble, des comprimés effervescents tamponnés, des comprimés à croquer, des sachets-dose, etc. ... les doses unitaires habituelles étant de l'ordre de 100 mg pour les formes pédiatriques et comprises entre 500 mg et 1 g pour les formes orales adultes.

Toutes ces formes galéniques présentent néanmoins des inconvénients. C'est le cas, par exemple, des formes à enrobage gastro-résistant et entérosoluble qui modifient la biodisponibilité du principe actif et de ses métabolites: le taux plasmatique n'est atteint que trois heures après la prise et la courbe des taux plasmatiques est étalée, ce qui a pour conséquence d'imposer, pour obtenir le même effet, une posologie plus forte que celle de l'ASA ordinaire. C'est le cas, également, des comprimés effervescents tamponnés car la solubilisation a des conséquences pharmacocinétiques importantes: d'une part, la résorption digestive est plus rapide et le pic plasmatique de salicylémie est atteint en moins d'une heure, deux fois plus vite qu'avec l'ASA ordinaire, d'autre part, l'élimination urinaire débute aussi plus rapidement. De plus, de telles formes impliquent l'incorporation de fortes doses d'éléments sodés pouvant être néfastes pour des nombreux sujets.

Quand à la DHE, il est connu que le traitement par la forme «gouttes» ou par la forme «comprimés» ne permet pas de satisfaire pleinement aux exigences prescriptives de certaines indications classiques de ce principe actif; c'est ainsi que, par exemple, pour les indications suivantes:
- migraines du matin,
- syndrôme d'hypotension orthostatique au lever,
- migraine hormonale à survenue nocturne,
- crampes et paresthésies nocturnes,

le traitement par la forme «gouttes» ou par la forme «comprimés» exigerait des administrations nocturnes répétées car la labilité de ces formes implique une élimination rapide du principe actif.

Pour pallier cette insuffisance d'emploi de ces deux formes galéniques de la DHE, il existe sur le marché pharmaceutique une nouvelle présentation galénique associant, sous forme de microgranules, de la DHE à libération immédiate et de la DHE à libération programmée, forme qui assure une action thérapeutique optimale durant les 12 heures qui suivent chaque prise médicamenteuse.

Ainsi les brevets FR-A-2 454 804 et FR-A-2 453 640 décrivent des préparations à effet immédiat-retard, dont les principes actifs sont respectivement la dihydroergotoxine et la dihydroergotamine. Par ailleurs, EP-A-64 485 décrit une préparation à effet retard dont le principe actif est l'acide acétylsalicylique et EP-A-36 350 concerne une composition à libération programmée d'un alcaloïde de l'ergot de seigle.

La présente invention concerne une composition médicamenteuse à base de dihydroergotamine et d'acide acétylsalicylique, caractérisée en ce qu'elle comprend:

a) des microgranules à libération immédiate formés chacun d'un grain support auquel est fixé un sel pharmaceutiquement acceptable de dihydroergotamine,

b) des microganules à libération programmée, formés chacun d'un grain support auquel est fixé un sel pharmaceutiquement acceptable de dihydroergotamine, enrobé d'un polymère de l'acide méthacrylique ou en polyméthacrylate,

c) des microgranules à libération programmée, formés chacun d'un grain support, recouvert d'un revêtement constitué d'un mélange contenant de l'acide acétylsalicylique, le revêtement étant protégé par un enrobage en polymère de l'acide méthacrylique,

et en ce que, en milieu artificiel, elle libère en 10 minutes de 30 à 35 p. 100 du poids de dihydroergotamine qu'elle contient, en 1 heure de 35 à 10 p. 100 du poids de dihydroergotamine et 4 p. 100 du poids de l'acide acétylsalicylique, en 4 heures de 55 à 75 p. 100 du poids de dihydroergotamine et 100 p. 100 du poids d'acide acétylsalicylique, et, en 8 heures 100 p. 100 du poids de dihydroergotamine qu'elle contient.

L'objet de la présente invention est une nouvelle composition pharmaceutique associant l'ASA à la DHE: l'ASA est présenté sous une nouvelle forme galénique qui permet une libération programmée du principe actif en milieu duodénal et qui pallie les inconvénients décrits ci-dessus, en particulier ceux liés à la tolérance gastrique; la DHE et ses sels pharmaceutiquement acceptables sont présentés sous la forme galénique à libération immédiate-retard qui pallie les insuffisances d'emploi des autres formes galéniques de ce principe actif. Une autre caractéristique de l'invention — qui est présentée sous forme de gélules — réside dans les doses contenues dans la composition pharmaceutique: 40 mg à 100 mg d'ASA sont en effet associés à 1,5 mg de DHE à libération immédiate et à 3,5 mg de DHE à libération programmée, mais les doses d'ASA peuvent être modifiées selon l'indication thérapeutique considérée.

Il est décrit, ci-après, la préparation de l'ASA suivant l'invention.

Cette préparation comporte un grain support en une matière non toxique, revêtu d'un revêtement en un mélange contenant de l'ASA, le revêtement étant enrobé d'un polymère d'acide méthacrylique. Le principe actif est libéré progressivement et régulièrement en milieu duodénal, pendant 4 heures. De ce fait, l'utilisation de l'ASA est améliorée et l'imprégnation médicamenteuse est la plus prolongée possible pour un minimum de prises tout au long du nycthémère, la libération programmée assurant un taux plasmatique stable, ce taux étant soutenu plus longtemps.

Pour réaliser en tel objectif, il est nécessaire que l'ASA ainsi programmé permette d'obtenir une cinétique de dissolution ayant des caractéristiques telles que, dans les conditions expérimentales choisies, c'est-à-dire en milieu aqueux artificiel, 4 p. 100 de l'ASA soient libérés en 1 heure; la cinétique de dissolution doit ensuite permettre d'obtenir, en 4 heures, la libération de 100 p. 100 du principe actif.

Pour obtenir les microgranules d'ASA à libération programmée, on utilise généralement la technique déjà décrite par la demanderesse dans le brevet français n° 2 454 804; on fixe le principe actif sur des grains support d'environ 500 à 600 µm de diamètre, ces derniers étant obtenus à partir d'une semence de saccharose et par enrobages successifs à l'aide d'une solution hydroalcoolique composée de saccharose de polyvidone-excipient, d'alcool industriel pharmaceutique et d'eau. On saupoudre à l'aide de talc et d'amidon de maïs. Après avoir laissé sécher, on calibre et on ne retient que les grains d'environ 500 à 600 µm de diamètre. On fixe ensuite l'ASA par poudrage et pulvérisation d'une solution hydroalcoolique de polyvidone-excipient et de phtalate d'éthyle. On laisse sécher, on calibre et on vérifie la teneur en principe actif. On enrobe les grains ainsi obtenus à l'aide d'une solution de polymère anionique de l'acide méthacrylique dans l'alcool industriel pharmaceutique: on saupoudre avec du talc et on recommence ces opérations jusqu'à ce que le programme de libération recherché corresponde à la cinétique de dissolution telle que décrite ci-dessus. Les microgranules ainsi terminés sont soumis à un nouveau contrôle analytique.

Il est donné, ci-après, à titre non limitatif, un exemple illustrant les quantités de produits nécessaires à la réalisation des gélules de l'invention.

On réalise les grains support dans les proportions suivantes, les quantités étant rapportées à 1 kg de grains support:

| Composition | Gamme générale grammes | Gamme préférée grammes |
|---|---|---|
| Saccharose | 300 à 750 | 694,6 |
| Amidon de maïs | 100 à 150 | 138,8 |
| Talc | 100 à 150 | 138,8 |
| Polyvidone-excipient | 25 à 30 | 27,8 |

les solvants intermédiaires étant l'alcool industriel pharmaceutique et l'eau. Après séchage et calibrage, les grains obtenus ont un diamètre de 500 microns environ.

Ces grains support sont ensuite enrobés à l'aide d'une solution hydroalcoolique de polyvidone-excipient et de phtalate d'éthyle contenant l'ASA, ce qui permet de fixer le principe actif sur les grains support de manière telle que pour 54 mg de microgranules, cette quantité titre 40 mg d'ASA. On laisse sécher, on calibre et on dose le principe actif de préférence par voie spectrophotométrique à 530 nm comparativement à une solution témoin d'ASA. On enrobe ces grains à l'aide d'une solution alcoolique de polymère anionique de l'acide méthacrylique; on poudre avec un talc dans la proportion d'environ 1/10 par rapport à la masse totale et on recommence les opérations jusqu'à ce que le programme de libération soit respecté. Les grains support et l'enrobage représentent environ 25 p. 100 du poids total.

La libération du principe actif devant être obtenue en milieu duodénal, la cinétique de libération in vitro est réalisée en milieu aqueux artificiel, à pH 6,9. Cette cinétique a permis d'obtenir les résultats suivants:

— après 1 heure        4 p. 100
— après 4 heures      100 p. 100

La présentation galénique de la DHE, dont la technique est dèjà décrite par la demanderesse dans le brevet français N° 2 453 639 permet une libération programmée du principe actif; il s'agit de microgranules d'action immédiate-retard ayant l'avantage de libérer immédiatement une dose de charge assez importante, la libération ultérieure de la DHE étant suffisamment régulière pendant 12 heures. De ce fait, l'utilisation du médicament est améliorée et la protection thérapeutique est ainsi obtenue tout au long du nycthémère, la libération programmée assurant un taux plasmatique stable, ce taux étant soutenu plus longtemps.

Pour réaliser un tel objectif, il est nécessaire que chaque préparation contienne à la fois des microgranules de DHE à libération immédiate et des microgranules de DHE à libération programmée, les proportions du mélange devant permettre d'obtenir une cinétique de dissolution ayant des caractéristiques telles que, dans les conditions expérimentales choisies, c'est-à-dire en milieux artificiels, 25 à 35% en poids de la totalité de la DHE contenue dans chaque préparation soient libérés en 10 minutes; la cinétique de dissolution permet ensuite d'obtenir, en 1 heure, une libération de 35 à 50% de DHE, la quantité de principe actif libérée atteignant 55 à 75 après 4 heures et 100% après 8 heures.

Pour obtenir les microgranules de DHE à libération immédiate, on peut fixer le principe actif sur des grains support d'environ 800 à 1000 µm de diamètre, les grains support étant obtenus à partir d'une semence de sucre et par enrobages successifs à l'aide d'un sirop de sucre chargé en amidon de maïs, en talc et en polyvidone-excipient, le sucre représentant notamment de 50 à 80% du poids du grain support, l'amidon de 10 à 25% de ce poids et le talc de 10 à 25% de ce poids, notamment pour les microgranules à libération programmée. A ces microgranules présentant la granulométrie désirée, la DHE est fixée sous forme d'une solution alcoolique. Les microgranules terminés seront étuvés puis soumis au dosage.

Pour obtenir les microgranules de DHE à libération programmée, on réalise les grains support dans les mêmes conditions que précédemment et on fixe la DHE en solution alcoolique. On enrobe ces grains à l'aide d'une solution alcoolique, telle qu'éthanolique, d'un polymère de l'acide méthacrylique, ou d'un poly(méthacrylate d'alcoyle inférieur), ces opérations de pulvérisation d'enrobage étant entrecoupées de poudrage au talc et de séchage à l'étuve. On peut aussi introduire dans un mélangeur un mélange d'acétone (5 parties) et d'isopropanol (2 parties) dans lequel on dissout de l'hydroxy propylméthylphtalate de cellulose. On ajoute une solution dans l'alcool isopropylique d'un polymérisat à base d'acide méthacrylique et de méthacrylate de méthyle. On ajoute la DHE et on met en suspension. Dans une turbine de dragéification, on introduit les grains support et on applique par pulvérisation sur ces derniers la suspension précédente. Après chaque étape d'enrobage, la vitesse de libération du principe actif est contrôlée et les différentes opérations sont poursuivies jusqu'à l'obtention du programme de libération désirée. La DHE fixée à chaque grain support représente de 200 à 800% du poids de ce grain. Les microgranules terminés sont à nouveau soumis à un contrôle.

Il est donné, ci-après, à titre non limitatif, un exemple illustrant la préparation.

Pour réaliser 300.000 doses de 5 mg chacune de DHE, dont 1,5 mg de principe actif est à libération immédiate et 3,5 mg à libération programmée, on prépare tout d'abord les microgranules de DHE à libération immédiate. On réalise les grains support ayant un diamètre compris entre 800 et 1000 µm avec environ 50 kg de saccharose, 10 kg d'amidon de maïs, 10 kg de talc et 12 kg de polyvidone-excipient. On y fixe 1,500 kg de DHE. On prépare ensuite les microgranules de DHE à libération programmée; c'est ainsi qu'une quantité déterminée des grains précédents à effet immédiat est enrobée à l'aide d'une solution de copolymérisat à base d'acrylates et de méthacrylates dans le phtalate d'éthyle et l'alcool pharmaceutique. On saupoudre avec du talc et on renouvelle les opérations jusqu'à ce que la cinétique de libération programmée soit respectée.

Les proportions des microgranules à libération immédiate et des microgranules à libération programmée sont définies en fonction de la cinétique de dissolution souhaitée, la dissolution en milieu artificiel libérant, en poids de DHE:

— après 10 minutes, de 30 à 35% et mieux 32% environ,

— après 1 heure, de 35 à 50% et mieux 45% environ,

— après 4 heures, de 55 à 75% et mieux 69%,

— après 8 heures, 100%.

Les microgranules à libération programmée d'ASA et ceux de DHE à libération immédiate et à libération programmée sont ensuite répartis dans des gélules de manière telle que chaque gélule contienne 40 mg d'ASA, 1,5 mg de DHE à libération immédiate et 3,5 mg de DHE à libération programmée (en abrégé ASA-40-DHE-5), le poids du contenu de chaque gélule étant de 300 mg environ. Ces titres en ASA et en DHE ne sont donnés qu'à titre illustratif non limitatif puisque des essais identiques ont été réalisés avec des quantités différentes de principes actifs.

*Essais toxicologiques*

Il a également été prcédé à l'étude toxicologique des gélules d'ASA-40-DHE-5 de l'invention comparativement à des gélules d'ASA-40-DHE-5 non programmées. Après pulvérisation du contenu des gélules et mise en suspension de la poudre ainsi obtenue dans une solution aqueuse de gomme arabique à 5 p. 100, cette suspension a été administrée par tubage gastrique à des souris de souche Swiss également réparties entre les deux sexes, chaque dose étudiée étant administrée à des lots de 5 mâles et de 5 femelles, le poids corporel des mâles étant compris entre 22 et 24 g celui des femelles entre 21 et 25 g. La dose maximum administrée a été de 5 gélules d'ASA-40-DHE-5 par kg de poids corporel, les animaux étant placés en observation pendant les 14 jours ayant suivi l'administration. Aucun symptôme d'intolérance n'a été observé; l'autopsie de 5 animaux mâles et de 5 animaux femelles s'est révélée particulièrement satisfaisante. Ainsi, nulle altération lésionnelle n'a été constatée.

D'autres essais ont été réalisés avec des rats de souche SPRAGUE-DAWLEY, dans les mêmes conditions que pour les souris. Les animaux mâles pesaient entre 170 et 220 g et les animaux femelles entre 150 et 170 g. La dose administrée a été identique à la précédente. Aucun symptôme d'intolérance n'a été observé pendant les 14 jours suivant l'administration et l'autopsie n'a rien révélé d'anormal sur le plan général et sur le plan histologique.

La tolérance chez l'animal a donc été la même, que la préparation en cause ait été ou non à libération programmée.

Des essais complémentaires toxicologiques ont été réalisés, chez la souris et chez le rat, avec des gélules titrant chacune 330 mg d'ASA à libération programmée ou non programmée, la quantité de DHE immédiat-retard, soit 5 mg/gélule, étant identique aux essais précédents.

Les résultats ont été satisfaisants et aucun symptôme d'intolérance n'a été observé pendant les 14 jours suivant l'administration.

Ces essais montrent donc la parfaite compatibilité, sur le plan de la toxicité, de la DHE et de l'ASA et de leur forme programmée.

*Essais de biodisponibilité et de tolérance gastrique*

Des essais ont été réalisés chez l'homme, pour d'une part connaître la biodisponibilité de l'ASA et de la DHE contenus dans la préparation de l'invention et, d'autre part, pour en connaître la tolérance gastrique.

1 — *Essais de biodisponibilité de l'ASA*

Pour les commodités de dosage et d'interprétation des résultats obtenus, les essais de biodisponibilité ont porté sur 2 préparations contenant 330 mg d'ASA par gélule:

préparation titrant 330 mg d'ASA à libération programmée par gélule (préparation A),

préparation de l'invention titrant par gélule 330 mg d'ASA à libération programmée, 1,5 mg de DHE à libération immédiate et 3,5 mg de DHE à libération programmée (préparation B).

La biodisponibilité a été appréciée après administration des deux préparations, chez l'homme, en cross-over, en mesurant les trois paramètres suivants:

— aires sous les courbes de concentration plasmatique,

— concentrations plasmatiques maxima obtenues (C max),

— temps des concentrations plasmatiques maxima (T max).

L'étude de biodisponibilité a été réalisée sur dix sujets de sexe masculin et féminin exempts d'affections cardio-vasculaires, hépatiques, rénales ou gastro-intestinales; ces sujets n'avaient reçu aucun médicament les jours précédents et pendant toute la durée de l'étude. Chaque sujet a reçu soit une gélule de préparation A, soit une gélule de préparation B; chacune des deux formulations a été absorbée au moins à 8 jours d'intervalle, avec 200 ml d'eau; enfin, chaque sujet entrant dans l'étude a reçu un numéro d'ordre correspondant à la randomisation de l'administration des deux formulations suivant quatre carrés latins. Les taux de la salicylémie — qui a été dosée suivant la méthode de ROWLAND et RIEGELMAN (J. Pharm. Sci., 1976, 56, 717-736) — ont été mesurés aux temps 0, 0,5, 1, 2, 3, 4, 6, 8, 10, 12 et 24 heures après d'administration.

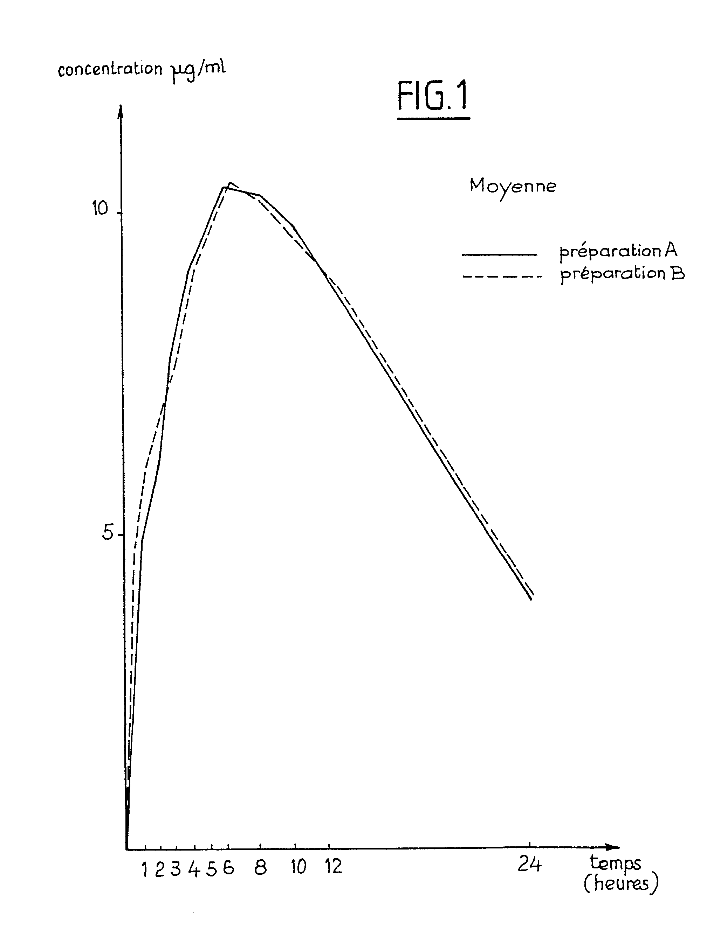

Les résultats moyens obtenus sont consignés dans le tableau ci-dessous, la figure 1 illustrant la concentration moyenne de la salicylémie ($\mu$g/ml) mesurée aux temps précités:

| Produits administrés | Aires sous les courbes | C max | T max |
|---|---|---|---|
| Préparation A | 129,39 ± 9,90 | 13,76 ± 0,95 | 7,00 ± 0,46 |
| Préparation B | 131,06 ± 9,70 | 13,92 ± 1,09 | 6,95 ± 0,34 |

Leur étude permet de constater que:

1 - les caractéristiques de biodisponibilité de l'ASA répondant effectivement aux critères d'une formulation retard,

2 - la présence de DHE dans la formulation de l'invention n'est pas incompatible avec la biodisponibilité de l'ASA telle que souhaitée pour une formulation retard.

2 — *Essais de biodisponibilité de la DHE*

Les essais de biodisponibilité ont porté sur les trois formulations suivantes:

. 2 gélules de la préparation de l'invention titrant, par gélule, 330 mg d'ASA à libération programmée, 1,5 mg de DHE à libération immédiate et 3,5 mg de DHE à libération programmée (préparation I),

. 2 gélules de la préparation de l'invention titrant, par gélule, 40 mg d'ASA à libération programmée, 1,5 mg de DHE à libération immédiate et 3,5 mg de DHE à libération programmée (préparation II).

. 2 gélules d'une préparation titrant, par gélule, 1,5 mg de DHE à libération immédiate et 3,5 mg de DHE à libération programmée (préparation III).

La biodisponibilité à été appréciée après administration des trois préparations, chez l'homme, en cross over, en mesurant les quatre paramètres suivants:

. concentrations plasmatiques moyennes,

. aires sous les courbes de concentration plasmatique,

. concentrations plasmatiques maxima obtenues (C max),

. temps des concentrations plasmatiques maxima (T max).

L'étude de biodisponibilité a été réalisée sur huit sujets masculins exempts d'affections cardio-vasculaires, hépatiques, rénales ou gastro-intestinales; ces sujets n'avaient reçu aucun médicament les jours précédents et pendant toute la durée de l'étude. Chaque sujet a reçu soit 2 gélules de la préparation I, soit 2 gélules de la préparation II, soit 2 gélules de la préparation III; chacune des trois formulations a été absorbée au moins à 8 jours d'intervalle, avec 200 ml d'eau; enfin, chaque sujet entrant dans l'étude a reçu un numéro d'ordre correspondant à la randomisation de l'administration des trois formulations suivant quatre carrés latins. Les taux de DHE contenus dans le plasma qui ont été dosés par chromatographie en phase gazeuse-spectrométrie de masse, ont été mesurés aux temps 0, 10 minutes, 20 minutes, 30 minutes, 45 minutes, 1 h, 2 h, 4 h, 6 h, 8 h, 10 h, 12 h et 24 heures après l'administration.

Les résultats moyens obtenus sont consignés dans les tableaux ci-après:

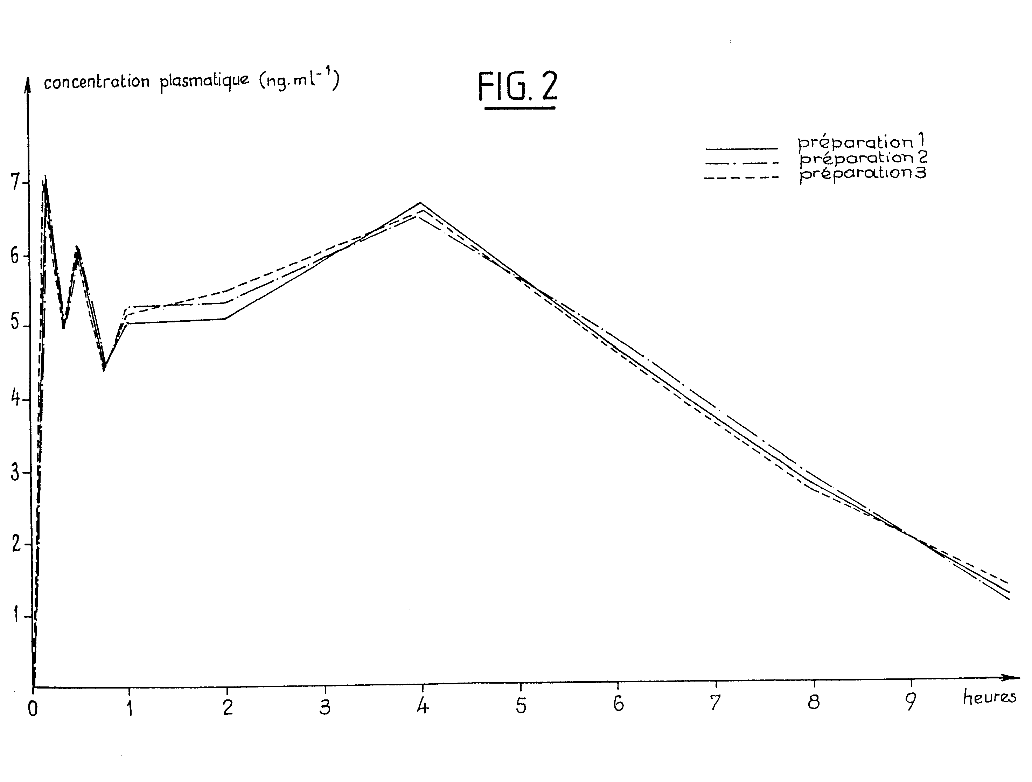

*Moyenne des concentrations plasmatiques de DHE (ng · ml⁻¹)*
(illustrée par la figure 2)

| Temps après administration | Préparation I | Préparation II | Préparation III |
|---|---|---|---|
| O | O | O | O |
| 10 min | 6,90 ± 2,02 | 6,92 ± 1,94 | 7,10 ± 1,96 |
| 20 min | 5,05 ± 1,25 | 5,01 ± 1,22 | 4,95 ± 1,20 |
| 30 min | 6,10 ± 1,40 | 6,08 ± 1,39 | 6,03 ± 1,36 |
| 45 min | 4,45 ± 1,02 | 4,55 ± 1,07 | 4,50 ± 1,06 |

| Temps après administration | Préparation I | Préparation II | Préparation III |
|---|---|---|---|
| 1 heure | 5,03 ± 1,00 | 5,17 ± 0,97 | 5,13 ± 0,94 |
| 2 heures | 5,12 ± 1,50 | 5,20 ± 1,44 | 5,28 ± 1,47 |
| 4 heures | 6,66 ± 1,07 | 6,49 ± 1,01 | 6,55 ± 1,02 |
| 6 heures | 4,62 ± 1,22 | 4,64 ± 1,17 | 4,59 ± 1,20 |
| 8 heures | 2,75 ± 1,17 | 2,78 ± 1,12 | 2,71 ± 1,15 |
| 10 heures | 1,19 ± 0,78 | 1,15 ± 0,75 | 1,20 ± 0,80 |
| 12 heures | 0 | 0 | 0 |
| 24 heures | 0 | 0 | 0 |

| Produits administrés | Aires sous les courbes | C max | T max |
|---|---|---|---|
| Préparation I | 43,12 ± 3,50 | 11,40 ± 3,35 | 2,12 ± 2,70 |
| Préparation II | 45,10 ± 3,77 | 11,15 ± 3,30 | 2,07 ± 2,60 |
| Préparation III | 44,51 ± 3,69 | 11,31 ± 3,41 | 2,02 ± 2,75 |

Les résultats obtenus permettent de constater que:

1. la préparation de l'invention possède toutes les caractéristiques de biodisponibilité répondant effectivement aux critères d'une formulation programmée,

2. la présence d'ASA, que cette dose soit faible ou soit importante, ne modifie pas la biodisponibilité de la DHE.

*Essai de tolérance gastrique*

L'étude de la tolérance gastrique a été réalisée par des essais contrôlés comparatifs en double aveugle avec répartition aléatoire entre la préparation de l'invention contenant 330 mg d'ASA et 5 mg de DHE et une préparation de référence contenant uniquement 330 mg d'ASA à libération programmée.

Les essais ont été conduits sur 55 sujets (21 hommes et 34 femmes) agés de 30 à 80 ans. 31 personnes ont reçu le produit de référence et 24 la préparation objet de l'invention.

Les deux préparations étaient présentées sous forme de gélules.

En fonction des indications thérapeutiques traitées (syndrome grippal, algies diverses, rééducation post-fracturaire, etc....), le nombre de gélules administrées a pu atteindre 60, le nombre de journées de traitement s'échelonnant de 2 à 9 jours ou plus.

Le tableau ci-après résume l'étude de la tolérance:

| Manifestations indésirables | Préparation de référence | Préparation de l'invention |
|---|---|---|
| Troubles gastriques importants | 0 | 0 |
| Troubles gastriques modérés | 2 | 2 |
| Troubles gastriques faibles | 5 | 4 |
| Troubles diarrhéiques faibles | 1 | 1 |

| Manifestations indésirables | Préparation de référence | Préparation de l'invention |
|---|---|---|
| Syndromes hémorragiques modérés (appréciés à l'hémocult) | 0 | 0 |
| Syndromes hémorragiques faibles (appréciés à l'hémocult) | 2 | 3 |
| TOTAUX | 10 | 10 |
| Aucune manifestation indésirable | 24 | 19 |

Il y a lieu de noter que chez aucun sujet il n'a été constaté de manifestations tégumentaires du type érythème, purpura, prurit, etc..., ou allergiques du type asthme ou rhinite. Les seuls manifestations indésirables ne sont donc survenus qu'au niveau de la sphère gastrointestinale ou de la sphère hémostatique. Ces intolérances peuvent se résumer comme suit:

| Manifestations indésirables chez un même sujet | Préparation de référence | Préparation de l'invention |
|---|---|---|
| à 3 niveaux (gastrique, intestinal, sanguin) | 0 | 0 p. 100 |
| à 2 niveaux (gastrique, intestinal, ou sanguin) | 9,67 p. 100 | 10,12 p. 100 |
| à 1 seul niveau (gastrique) | 19,20 p. 100 | 20,07 p. 100 |
| TOTAUX | 22,58 p. 100 (7 cas) | 23,12 p. 100 (5 cas) |

Ainsi donc, avec la préparation de référence, 24 cas, soit 77,41 p. 100, n'ont pas présenté de manifestation indésirable et avec la préparation de l'invention, 19 cas, soit 79,17 p. 100 n'ont pas présenté de manifestation indésirable.

Il apparaît ainsi que la préparation de l'invention est aussi bien tolérée que la préparation de référence, ce qui implique que la présence de DHE dans la formulation de l'invention n'apporte aucune modification à la bonne tolérance de l'ASA à libération programmée.

*Essais cliniques*

Comme précisé ci-dessus, des travaux cliniques réalisés par ZECKERT et al. (Haemostasis, 1982, 11, 149-153) ont associé des comprimés de DHE non programmés à des comprimés d'ASA non programmés, dans la prévention des thromboembolies.

D'autres travaux cliniques ont également montré que sur différénts types de migraines, celui de la «migraine dite du matin», c'est-à-dire survenant dès le réveil, était très sensible à la DHE présentée sous forme de gélules et contenant 1,5 mg de DHE à libération immédiate et 3,5 mg de DHE à libération programmée, ce résultat étant dû à une action prolongée et continue du principe actif sur le nycthémère.

Néanmoins, les résultats positifs ne représentent qu'environ 50 p. 100 des cas cliniques traités.

Il a donc semblé intéressant d'étudier le rôle de l'ASA dans la formulation de l'invention et pour cela, des essais ciliques ont été réalisés sur 30 sujets adultes (5 hommes et 25 femmes) âgés entre 25 ans et 52 ans et plus. Ces essais ont été pratiqués avec les préparations suivantes:

. préparation P1: gélules de DHE titrant 1,5 mg de DHE à libération immédiate et 3,5 mg de DHE à libération programmée (médicament de référence),

. préparation P2: gélules contenant 1,5 mg de DHE à libération immédiate et 3,5 mg de DHE à libération programmée + 40 mg d'ASA non programmé,

. préparation P3: gélules contenant 1,5 mg de DHE à libération immédiate et 3,5 mg de DHE à libération programmée + 40 mg d'ASA à libération programmée. Cette préparation correspond à la médication de l'invention.

La posologie a été, aussi bien pour P1 et P2 que pour P3, de 2 gélules par jour (1 le matin et 1 le soir).

Pour un même sujet et à chaque administration, il a été prescrit:

*1° séquence de 2 mois:* soit une gélule P3, soit une gélule P2,

*2° séquence de 2 mois:* soit une gélule P2, soit une gélule P1,

*3° séquence de 2 mois:* soit une gélule P2, soit une gélule P1.

Parmi les critères d'efficacité a été retenu le nombre de crises migraineuses du matin. C'est ainsi que, sur 782 crises dénombrées, elles ont été ramenées à:
— 367, avec P1,
soit une amélioration de 53,06 p. 100
— 332, avec P2,
soit une amélioration de 57,54 p. 100
— 280, avec P3,
soit une amélioration de 64,19 p. 100.

On peut en déduire que, si la présence d'ASA non programmée dans P2 permet d'augmenter le pourcentage d'amélioration de 53,06 p. 100 à 57,54 p. 100 (soit une augmentation de 8,44 p. 100), la présence d'ASA programmée dans la préparation P3 (correspondant à la formulation de l'invention permet d'augmenter le pourcentage d'amélioration de 53,06 p. 100 à 64,19 p. 100 (soit une augmentation de 20,97 p. 100 par rapport à P1 et de 11,56 p. 100 par rapport à P2).

Il apparaît donc que le médicament de l'invention permet une diminution des crises migraineuses du matin, cette diminution étant plus conséquente quand l'ASA contenu dans l'association est présenté sous la forme d'une préparation programmée.

Il est donné ci-dessous, à titre d'exemple non limitatif, une formulation unitaire du médicament de l'invention:

| | | |
|---|---|---|
| Dihydroergotamine méthanesulfonate à libération immédiate | 1,5 | mg |
| Dihydroergotamine méthanesulfonate à libération programmée | 3,5 | mg |
| Acide acétylsalicylique | 40 | mg |
| Saccharose | 80 | mg |
| Amidon de maïs | 22 | mg |
| Talc | 45 | mg |
| Lactose | 21,5 | mg |
| Polyvidone-excipient | 72 | mg |
| Polymère anionique de l'acide méthacrylique | 11 | mg |
| Phtalate d'éthyle | 3,5 | mg |

q.s.p. 1 gélule terminée à 340 mg environ

Par son activité régulatrice sur le tonus vasculaire, caractérisée sur le plan pharmacologique par une action sympatholytique de type adrénolytique α, par une action antisérotonique et par une action tonique veineuse, le médicament de l'invention est indiqué dans le traitement de fond de la maladie migraineuse (migraine du réveil, migraine digestive, migraine ophtalmique, migraine liée à la prise de contraceptifs oraux, migraines cataméniales, algies vasculaires de la face) dans l'insuffisance veineuse des membres inférieurs (jambes lourdes, oedèmes de la fin de journée, varices primitives) et, dans les troubles survenant au cours des traitements par les neuroleptiques et les psychotropes (hypotension orthostatique, effets atropiniques). Dans ces indications, la posologie usuelle du médicament de l'invention est de 2 gélules par jour à administrer 1 le matin et 1 le soir; le médicament est avantageusement composé de 1,5 mg de DHE à libération immédiate, de 3,5 mg de DHE à libération programmée associés à de l'ASA à libération programmée. Le titre en ASA peut avantageusement varier entre 30 et 500 mg par dose unitaire, les titres préférés étant de 40 mg par prise ou de 330 mg par prise selon l'indication thérapeutique considérée mais ces doses ne sont données qu'à titre illustratif non limitatif.

**Revendications pour les Etats contractants:**
BE, CH, DE, FR, GB, IT,LI, LU

1. Composition médicamenteuse à base de dihydroergotamine et d'acide acétylsalicylique, caractérisée en ce qu'elle comprend:

a) des microgranules à libération immédiate formés chacun d'un grain support auquel est fixé un sel pharmaceutiquement acceptable de dihydroergotamine,

b) des microgranules à libération programmée, formés chacun d'un grain support auquel est fixé un sel pharmaceutiquement acceptable de dihydroergotamine, enrobé d'un polymère de l'acide méthacrylique ou en polyméthacrylate,

c) des microgranules à libération programmée, formés chacun d'un grain support, recouvert d'un revêtement constitué d'un mélange contenant de l'acide acétylsalicylique, le revêtement étant protégé par un enrobage en polymère de l'acide méthacrylique,

et en ce que, en milieu artificiel, elle libère en 10 minutes de 30 à 35 p. 100 du poids de dihydroergotamine qu'elle contient, en 1 heure de 35 à 50 p. 100 du poids de dihydroergotamine et 4 p. 100 du poids de l'acide acétylsalicylique, en 4 heures de 55 à 75 p. 100 du poids de dihydroergotamine et 100 p. 100 du poids d'acide acétylsalicylique, et, en 8 heures 100 p. 100 du poids de dihydroergotamine qu'elle contient.

2. Composition médicamenteuse suivant la revendication 1, caractérisée en ce qu'en milieu artificiel, elle libère en 10 minutes 32 p. 100 environ du poids de dihydroergotamine qu'elle contient, en 1 heure 45 p. 100 environ, et en 4 heures 69 p. 100 environ.

3. Copmposition médicamenteuse suivant la revendication 1 ou 2, caractérisée en ce qu'elle est présentée sous forme de gélules.

4. Composition médicamenteuse suivant l'une des revendications 1 à 3, caractérisée en ce qu'elle contient:
— 1,5 mg der dihydroergotamine à libération immédiate,
— 3,5 mg de dihydroergotamine à libération programmée.

5. Composition médicamenteuse suivant l'une des revendications 1 à 4, caractérisée en ce qu'elle contient de 30 à 500 mg d'acide acétylsalicylique à libération programmée.

**Revendications pour l'Etat contractant:** AT

1. Procédé de préparation d'une composition médicamenteuse à base de dihydroergotamine et d'acide acétylsalicylique, caractérisé en ce que l'on prépare:

a) des microgranules à libération immédiate formés chacun d'un grain support auquel est fixé un sel pharmaceutiquement acceptable de didydroergotamine,

b) des microgranules à libération programmée, formés chacun d'un grain support auquel est fixé un sel pharmaceutiquement acceptable de dihydroergotamine, enrobé d'un polymère de l'acide méthacrylique ou en polyméthacrylate,

c) des microgranules à libération programmée, formés chacun d'un grain support, recouvert d'un revêtement constitué d'un mélange contenant de l'acide acétylsalicylique, le revêtement étant protégé par un enrobage en polymère de l'acide méthacrylique,

en en ce que, en milieu artificiel, la composition libère en 10 minutes de 30 à 35 p. 100 du poids de dihydroergotamine qu'elle contient, en 1 heure de 35 à 50 p. 100 du poids de dihydroergotamine et 4 p. 100 du poids de l'acide acétylsalicylique, en 4 heures de 55 à 75 p. 100 du poids de dihydroergotamine et 100 p. 100 du poids d'acide acétylsalicylique, et, en 8 heures 100 p. 100 du poids de dihydroergotamine qu'elle contient.

2. Procédé suivant la revendication 1, caractérisé en ce qu'en milieu artificiel, la composition libère en 10 minutes 32 p. 100 environ du poids de dihydroergotamine qu'elle contient, en 1 heure 45 p. 100 environ, et en 4 heures 69 p. 100 environ.

3. Procédé suivant le revendication 1 ou 2, caractérisé en ce que la composition est présentée sous forme de gélules.

4. Procédé suivant l'une des revendications 1 à 3, caractérisé en ce que la composition contient:
— 1,5 mg de dihydroergotamine à libération immédiate,
— 3,5 mg de dihydroergotamine à libération programmée.

5. Procédé suivant l'une des revendications 1 à 4, caractérisé en ce que la composition contient de 30 à 500 mg d'acide acétylsalicylique à libération programmée.

**Patentansprüche für die Vertragsstaaten:**
BE, CH, DE, FR, GB, IT, LI, LU

1. Medikamentöse Zubereitung auf Basis von Dihydroergotamin und Acetylsalicylsäure, dadurch gekennzeichnet, dass sie umfasst:

a) Mikrogranulat mit sofortiger Abgabe, gebildet jeweils aus einem Trägerkorn, an das ein pharmazeutisch unbedenkliches Salz von Dihydroergotamin fixiert ist,

b) Mikrogranulat mit programmierter Abgabe, gebildet jeweils aus einem Trägerkorn, an das ein pharmazeutisch unbedenkliches Salz von Dihydroergotamin fixiert ist und das von einem Polymerisat von Methacrylsäure oder Polymethacrylat überzogen ist,

c) Mikrogranulat mit programmierter Abgabe, gebildet jeweils aus einem Trägerkorn, das von einer Umhüllung bedeckt ist, die aus einem Acetylsalicylsäure enthaltenden Gemisch gebildet ist und von einer Umhüllung aus Methacrylsäurepolymerisat geschützt ist,

und dass sie in einem künstlichen Medium in 10 Minuten 30 bis 35 Gew.-% des in ihr enthaltenen Dihydroergotamins, in 1 Stunde 35 bis 50 Gew.-% des Dihydroergotamins und 4% des Gewichts der Acetylsalicylsäure, in 4 Stunden 55 bis 75% des Gewichts des Dihydroergotamins und 100% des Gewichts der Acetylsalicylsäure und in 8 Stunden 100% des Gewichts des in ihr enthaltenen Dihydroergotamins abgibt.

2. Medikamentöse Zubereitung nach Anspruch 1, dadurch gekennzeichnet, dass sie in einem künstlichen Medium in 10 Minuten etwa 32% des Gewichts des darin enthaltenen Dihydroergotamins, in 1 Stunde etwa 45% und in 4 Stunden etwa 69% abgibt.

3. Medikamentöse Zubereitung nach Anspruch 1 oder 2, dadurch gekennzeichnet, dass sie in Form von Gelatinekapseln dargeboten wird.

4. Medikamentöse Zubereitung nach einem der Ansprüche 1 bis 3, dadurch gekennzeichnet, dass sie 1,5 mg Dihydroergotamin mit sofortiger Abgabe und 3,5 mg Dihydroergotamin mit programmierter Abgabe enthält.

5. Medikamentöse Zubereitung nach einem der Ansprüche 1 bis 4, dadurch gekennzeichnet, dass sie 30 bis 500 mg Acetylsalicylsäure mit programmierter Abgabe enthält.

**Patentansprüche für den Vertragsstaat: AT**

1. Verfahren zur Herstellung einer medikamentösen Zubereitung auf Basis von Dihydroergotamin und Acetylsalicylsäure, dadurch gekennzeichnet, dass man herstellt

a) Mikrogranulat mit sofortiger Abgabe, gebildet jeweils aus einem Trägerkorn, an das ein pharmazeutisch unbedenkliches Salz von Dihydroergotamin fixiert ist,

b) Mikrogranulat mit programmierter Abgabe, gebildet jeweils aus einem Trägerkorn, an das ein pharmazeutisch unbedenkliches Salz von Dihydroergotamin fixiert und das von einem Polymerisat von Methacrylsäure oder Polymethacrylat umhüllt ist,

c) Mikrogranulat mit programmierter Abgabe, gebildet jeweils aus einem Trägerkorn, das von einem Überzug, der aus einem Acetylsalicylsäure enthaltenden Gemisch besteht und durch eine Umhüllung aus einem Polymerisat von Methacrylsäure geschützt ist, bedeckt ist,
und dass die Zubereitung in einem künstlichen Medium in 10 Minuten 30 bis 35 Gew.-% des Gewichts des darin enthaltenen Dihydroergotamins, in 1 Stunde 35 bis 50% des Gewichts des Dihydroergotamins und 4% des Gewichts der Acetylsalicylsäure, in 4 Stunden 55 bis 75% des Gewichts des Dihydroergotamins und 100% des Gewichts der Acetylsalicylsäure und in 8 Stunden 100% des Gewichts des darin enthaltenen Dihydroergotamins abgibt.

2. Verfahren nach Anspruch 1, dadurch gekennzeichnet, dass die Zubereitung in einem künstlichen Medium in 10 Minuten etwa 32% des Gewichts des darin enthaltenen Dihydroergotamins, in 1 Stunde etwa 45% und in 4 Stunden etwa 69% abgibt.

3. Verfahren nach Anspruch 1 oder 2, dadurch gekennzeichnet, dass die Zubereitung in Form von Gelatinekapseln dargeboten wird.

4. Verfahren nach einem der Ansprüche 1 bis 3, dadurch gekennzeichnet, dass die Zubereitung 1,5 mg Dihydroergotamin mit sofortiger Abgabe und 3,5 mg Dihydroergotamin mit programmierter Abgabe enthält.

5. Verfahren nach einem der Ansprüche 1 bis 4, dadurch gekennzeichnet, dass die Zubereitung 30 bis 500 mg Acetylsalicylsäure mit programmierter Abgabe enthält.

**Claims for the Contracting States:**
BE, CH, DE, FR, GB, IT, LI, LU

1. A dihydroergotamine and acetylsalicylic acid based medical composition, characterized in that it comprises:

a) microgranules for immediate release each formed by a carrier particle to which a pharmaceutically acceptable salt of dihydroergotamine is fixed,

b) microgranules for controlled release, each formed by a carrier particle to which a pharmaceutically acceptable salt of dihydroergotamine is fixed, which is coated with a polymer of methacrylic acid or polymethacrylate,

c) microgranules for controlled release, each formed of a carrier particle, covered with a coating consisting of a mixture containing acetylsalicylic acid, the coating being protected by a layer from methacrylic acid,
and in that, in artificial medium, it releases 30 to 35% of the weight of dihydroergotamine that it contains within 10 minutes, 35 to 50% of the weight of dihydroergotamine and 4% of the weight of acetylsalicylic acid within one hour, 55 to 75% of the weight of dihydroergotamine and 100% of the weight of acetylsalicylic acid within four hours, and 100% of the weight of dihydroergotamine that it contains within eight hours.

2. Medicinal composition according to claim 1, characterized in that, in artificial medium, it releases about 32% of the weight of dihydroergotamine that it contains, about 45% within one hour and about 69% within four hours.

3. Medicinal composition according to claim 1 or 2, characterized in that it is presented in the form of capsules.

4. Medicinal composition according to any of claims 1 to 3, characterized in that it contains:
— 1.5 mg of dihydroergotamine for immediate release,
— 3.5 mg of dihydroergotamine for controlled release.

5. Medicinal composition according to any of claims 1 to 4, characterized in that it contains from 30 to 500 mg of acetylsalicylic acid for controlled release.

**Claims for the Contracting State: AT**

1. Process for the preparation of a dihydroergotamine and acetylsalicylic acid based medicinal composition, characterized in that there are prepared:

a) microgranules for immediate release each formed by a carrier particle to which a pharmaceutically acceptable salt of dihydroergotamine is fixed,

b) microgranules for controlled release, each formed by a carrier particle to which a pharmaceutically acceptable salt of dihydroergotamine is fixed, which is coated with a polymer of methacrylic acid or polymethacrylate,

c) microgranules for controlled release, each formed of a carrier particle, covered with a coating consisting of a mixture containing acetylsalicylic acid, the coating being protected by a layer from methacrylic acid,

and in that, in artificial medium, it releases 30 to 35% of the weight of dihydroergotamine that it contains within 10 minutes, 35 to 50% of the weight of dihydroergotamine and 4% of the weight of acetylsalicylic acid within one hour, 55 to 75% of the weight of dihydroergotamine and 100% of the weight of acetylsalicylic acid within four hours, and 100% of the weight of dihydroergotamine that it contains within eight hours.

2. Process according to claim 1, characterized in that, in artificial medium, the composition releases about 32% of the weight of dihydroergotamine that it contains, about 45% within one hour and about 69% within four hours.

3. Process according to claim 1 or 2, characterized in that the composition is presented in the form of capsules.

4. Process according to any of claims 1 to 3, characterized in that the composition contains:
— 1.5 mg of dihydroergotamine for immediate release,
— 3.5 mg of dihydroergotamine for controlled release.

5. Process according to any of claims 1 to 4, characterised in that the composition contains from 30 to 500 mg of acetylsalicylic acid for controlled release.

concentration μg/ml

FIG.1

Moyenne

——— préparation A
- - - - préparation B

concentration plasmatique (ng.ml$^{-1}$)

FIG. 2

préparation 1
préparation 2
préparation 3

0 117 164

heures